# FASCICULE DE BREVET EUROPEEN

(11) **EP 3 145 590 B1**
(45) Date de publication et mention de la délivrance du brevet: **24.04.2024**
(21) Numéro de dépôt: 15723507.8
(22) Date de dépôt: 19.05.2015
(51) Int. Cl.: A61Q 1/12, A61K 8/58, A61K 8/894, A61K 8/04

(54) **COMPOSITION COSMÉTIQUE DE SOIN ET/OU DE MAQUILLAGE DE LA PEAU SOUS LA FORME D'UNE ÉMULSION DE TYPE EAU-DANS-SILICONE**
KOSMETISCHE ZUSAMMENSETZUNG ZUR PFLEGE UND/ODER ZUM SCHMINKEN DER HAUT IN FORM EINER EMULSION VOM WASSER-IN-SILIKON-TYP
COSMETIC COMPOSITION FOR CARING FOR AND/OR FOR MAKING UP THE SKIN, IN THE FORM OF AN EMULSION OF THE WATER-IN-SILICONE TYPE

(30) Priorité: 20.05.2014 FR 1454492
(43) Date de publication de la demande: 29.03.2017
(73) Titulaire: The Boots Company PLC, Nottingham NG2 3AA (GB)
(72) Inventeur: GUILLAUME, Bruno, F-35500 Vitre (FR)
(74) Mandataire: Cabinet Le Guen Maillet
(86) Numéro de dépôt international: PCT/EP2015/060957
(87) Numéro de publication internationale: WO 2015/177118

(56) Documents cités:
- DE-A1- 10 223 694
- FR-A1- 2 894 467
- US-A1- 2005 142 094
- US-A1- 2012 107 379

## Description

La présente invention concerne une composition cosmétique de soin et/ou de maquillage de la peau composée d'une émulsion du type eau-dans-silicone expansée à l'aide de bulles gazeuses nanométriques.

La peau est l'organe majoritaire chez l'homme avec une surface et une masse inégalées. Sa structure particulière composée de différentes couches de tissus en fait une protection essentielle du corps humain.

L'épiderme est la couche externe de la peau, et sa partie la plus superficielle est constituée de cellules appelées cornéocytes. Ce sont des cellules kératinisées qui se détachent du Stratum corneum et desquament chaque jour. Ainsi, la couche externe de la peau comporte des irrégularités dues aux cellules ayant quitté l'organisme. De même que les cornéocytes, les espaces qu'ils laissent en desquamant ont une épaisseur comprise entre 0,1 et 1 µm.

Comme expliqué ci-dessus, l'épiderme n'a pas une surface lisse et uniforme. Classiquement, les crèmes cosmétiques ne tiennent pas compte de ces aspérités et offrent donc un recouvrement irrégulier à la peau.

De plus, ce type de composition peut comprendre des pigments. Afin d'augmenter leur affinité avec la phase dispersante du cosmétique, les pigments sont traditionnellement traités en surface avant dispersion. Cependant, les forces de cisaillement appliquées sur ces pigments lors du traitement de la phase dispersante engendrent des coupures sur les pigments, exposant ainsi une partie non traitée au reste des compositions. Cela se traduit par une mauvaise dispersion des pigments.

La société demanderesse a cherché à résoudre ces problèmes et, suite à ses recherches, a mis au point une formulation soin et/ou de maquillage dans laquelle une dispersion de gouttelettes de gaz de diamètre de l'ordre du nanomètre est ajoutée. Elle a, en effet, constaté que des gouttelettes de cette taille permettaient, une fois la composition appliquée sur la peau, de combler les espaces entre les cellules ou les autres irrégularités observées à la surface de la peau. De plus, le gaz contenu dans les gouttelettes étant hydrophobe, celles-ci vont venir tapisser la surface non traitée des pigments sectionnés et augmenter ainsi leur affinité pour la phase dispersante tout en empêchant leur regroupement.

A titre de compositions foisonnées destinées à être appliquées sur la peau, on connaît par exemple la demande de brevet EP 1 046 387 qui concerne une composition cosmétique foisonnée comprenant une émulsion huile-dans-eau et des bulles gazeuses. Il est divulgué dans cette demande de brevet que les bulles gazeuses ont un diamètre moyen compris entre 20 µm et 500 µm, ce qui est très supérieur à la taille des creux de la surface de l'épiderme tels que mentionnés précédemment. De plus, ce document se réfère à une émulsion du type huile-dans-eau uniquement.

La demande de brevet FR 2 850 017 concerne une composition cosmétique sous forme de mousse comprenant une émulsion huile-dans-eau expansée à l'aide d'un gaz. Le document se réfère aux mousses comme ayant des bulles de diamètre compris entre 10 et 50 µm, ce qui est d'une taille bien supérieure aux creux laissés par les cornéocytes lors de la desquamation et n'a donc pas de rôle de comblement. De plus, cette demande de brevet concerne des fonds de teint du type huile-dans-eau umquement. US2012107379 divulgue des compositions cosmétique à base d'eau sous forme de mousse.

Les compositions de maquillage de la peau telles que les fonds de teint les plus utilisés aujourd'hui sont de type émulsion eau-dans-silicone. Or, l'air est silicophobe autant qu'hydrophobe. Il est donc difficile d'envisager d'introduire de l'air dans de telles compositions et les informations divulguées dans l'état de la technique tel que celui cité précédemment s'avèrent inadaptées à de telles formulations.

Un but de l'invention est de pallier les inconvénients pré-cités. En particulier, un des buts de l'invention est de proposer une composition de soin et/ou de maquillage de la peau qui, une fois appliquée sur la peau, recouvre efficacement les aspérités épidermiques.

Un autre but de l'invention est de proposer une telle composition dans laquelle les pigments sont dispersés de manière homogène.

Un autre but de l'invention est de proposer une telle composition qui présente de très bonnes qualités en termes de tenue et de durabilité une fois appliquée sur la peau, une remarquable facilité d'application ainsi qu'une excellente adhésion à la surface d'application.

On entend par tenue la résistance au temps du cosmétique.

A cet effet, la présente invention concerne une composition cosmétique de soin et/ou de maquillage de la peau, se présentant sous la forme d'une émulsion de type eau-dans-silicone, de densité inférieure à 1,1, et comprenant, en poids, par rapport au poids de la composition :
- entre 40 et 90 % d'une phase grasse siliconée continue comprenant au moins une huile siliconée et entre 1 et 4% de cetyl PEG/PPG-10/1 diméthicone et entre 1 et 6% de cyclopentasiloxane et PEG/ PPG- 19/19 diméthicone, à titre de tensioactifs siliconés de HLB inférieur ou égal à 10,
- d'une phase aqueuse dispersée dans ladite phase grasse continue,
- une dispersion de gouttelettes de gaz de diamètre moyen compris entre 10 nm et 11nm,
- un système stabilisant pour stabiliser la dispersion de gouttelettes de gaz comprenant entre 0,8 et 5% de PEG-150 distéarate à titre de tensioactif de HLB supérieur ou égal à 16 et entre 0,01 et 1% de polyacrylate de sodium à titre de composé gélifiant hydrophile.

On entend par composition cosmétique de maquillage ou de soin de la peau toute formulation cosmétique ayant pour but d'être appliquée sur la peau. La composition cosmétique visée par la présente invention peut prendre la forme d'un produit de maquillage, d'une crème ou plus préférentiellement d'un fond de teint.

On entend par émulsions de type eau-dans-silicone, toute émulsion dont la phase continue ou dispersante est composée de matières grasses telles que des huiles, des cires, des lipides, des acides gras et dont le solvant présent en quantité majoritaire dans cette phase contient une substance siliconée, et la phase discontinue ou dispersée contient de l'eau ou un solvant aqueux comme la glycérine.

On entend par huile de silicone, une huile comprenant au moins un atome de silicium, et notamment au moins un groupe Si-O.

On entend par « ≥ » et « ≤ », « supérieur(e) ou égal(e) » et « inférieur(e) ou égal(e) », respectivement.

On entend par HLB (de l'anglais « Hydrophilic Lipophilic Balance »), la balance Hydrophile-Lipophile caractérisant la solubilité d'un composé tensioactif. On attribue à chaque tensioactif une valeur de HLB comprise entre 1 et 20. Cette échelle est l'échelle de Griffin définie l'ouvrage J. Soc. Cosm. Chem., 1954 (Volume 5), pages 249-256. Cette échelle permet d'apprécier le caractère plutôt lipophile ou au contraire plutôt hydrophile d'un composé amphiphile. La valeur HLB est obtenue par le calcul suivant :
HLB = 20 * (Mh/M) où Mh est la masse moléculaire de la partie hydrophile et MM est la masse moléculaire de la molécule dans son entier.

La densité indiquée est le rapport de la masse volumique de l'émulsion selon l'invention par rapport à celle d'un même volume d'eau.

Sauf indication contraire, les pourcentages sont exprimés en poids, par rapport au poids total de la composition.

La composition selon l'invention contient ainsi des gouttelettes de gaz de taille nanométrique, par exemple, des gouttelettes d'air se trouvant dans la composition sous la forme d'une dispersion ou d'une émulsion. Lors de l'application de la composition cosmétique sur la peau, les gouttelettes nanométriques vont pouvoir s'insérer entre les cornéocytes et les creux à la surface de la peau, avec pour résultat l'obtention d'une surface lisse et continue, améliorant ainsi le rendu et la durabilité de la tenue du produit.

Préférentiellement, les gouttelettes de gaz présentent un diamètre moyen compris entre 50 nm et 800 nm, plus préférentiellement entre 100 et 800 nm. Généralement, les gouttelettes de gaz présentent une uniformité au niveau de leur taille. Néanmoins, l'on peut considérer qu'entre 70 et 100 %, préférentiellement entre 90 et 100 %, en nombre, des gouttelettes de gaz présentent les caractéristiques de diamètre indiquées dans la présente demande. Le diamètre des gouttelettes peut être mesuré dans la composition avant son application au moyen d'un microscope électronique par transmission (MET).

Préférentiellement, le gaz est choisi parmi l'air, l'argon, le dioxyde de carbone ou l'azote. Dans un mode de réalisation préféré de l'invention, les gouttelettes sont des gouttelettes de dioxyde de carbone.

Avantageusement, la dispersion de gouttelettes de gaz se présente sous la forme d'une dispersion gaz-dans-eau-dans silicone.

La composition selon l'invention comprend ainsi une première émulsion d'eau, ou de solvant aqueux, dans une phase grasse et seconde émulsion sous forme de gouttes d'eau refermant chacune une gouttelette de gaz, cette seconde émulsion étant également dispersée dans la phase grasse. Les gouttelettes de gaz sont donc entourées d'un film d'eau. Cette émulsion d'eau, renfermant du gaz, dans la phase grasse peut encore être considérée comme une phase mousse.

Préférentiellement, le tensioactif siliconé présente un HLB compris entre 2 et 7.

Les tensioactifs siliconés de HLB ≤ 10, à la fois du Cetyl PEG/PPG-10/1 diméthicone et du cyclopentasiloxane et PEG/PPG- 19/19 diméthicone.

La composition comprend entre 0,1 et 8 % de tensioactif siliconé de HLB≤10.

La phase grasse comprend essentiellement à titre de solvant une huile de silicone. Comme huile siliconée utilisable dans l'invention, on peut citer les huiles de silicones linéaires, ramifiées ou cycliques ayant notamment de 2 à 7 atomes de silicium, ces silicones comportant éventuellement des groupes alkyle ou alkoxy ayant de 1 à 10 atomes de carbone pendants ou au bout de chaque silicone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthylhexyl trisiloxane, l'heptaméthyloctyl trisiloxane, l'hexaméthyl disiloxané, l'octaméthyl trisiloxane, le décaméthyl tétrasiloxane, le dodécaméthyl pentasiloxane et leurs mélanges.

La phase grasse selon l'invention peut comprendre, de plus, des huiles hydrocarbonées, fluorées ou non, ou leurs mélanges. Les huiles peuvent être volatiles ou non volatiles, d'origine animale, végétale, minérale ou synthétique. Préférentiellement, ces huiles sont présentes en quantité inférieure à celle de l'huile siliconée.

La phase grasse est entre 40 et 90 % de la composition, en poids.

Selon un mode de réalisation de l'invention, la composition comprend un système stabilisant pour stabiliser la dispersion de gouttelettes de gaz comprenant au moins un tensioactif de HLB ≥ 16, préférentiellement de HLB ≥ 18, et/ou au moins un composé gélifiant hydrophile. Le tensioactif de HLB élevé vient se placer à l'interface gaz/eau, à l'intérieur des gouttelettes d'eau de la phase mousse.

Le tensioactif de HLB ≥ à 16, le PEG-150 Distearate. Préférentiellement, la composition comprend entre 0,001 et 2% dudit tensioactif de HLB supérieur ou égal à 16, préférentiellement supérieur à 18.

Le système stabilisant comprend, à titre de gélifiant hydrophile, le polyacrylate de sodium. Le système stabilisant est présent à hauteur de 0,01 à1 % en poids, par rapport au poids total de la composition.

Selon un mode de réalisation préféré de l'invention, le système stabilisant comprend, à titre de gélifiant hydrophile, au moins un polymère thermo-gélifiant. A ce titre, on se reportera au brevet européen n° EP 1 521 795 qui décrit un tel polymère et qui spécifie ce que l'on entend par « polymère thermo-gélifiant » : Il s'agit d'un polymère formé d'unités hydrophobes, thermosensibles, et d'unité hydrophiles. La formation de gel est expliquée par l'auto-association des portions thermosensibles en micro-domaines hydrophobes; l'ensemble du polymère étant maintenu en solution par les segments hydrophiles. Les propriétés de viscosification et celles du gel sont alors contrôlées par la longueur respective des différents segments et par le ratio hydrophobe/hydrophile (L.E. Bromberg, Adv. Drug Delivery Reviews 31 (1998) 197-221). Plus précisément, le polymère thermogélifiant est un polymère qui, à faible concentration dans l'eau, se présente sous forme liquide (faible viscosité) à une température ambiante comprise généralement entre 18 et 25°C et qui gélifie à la température corporelle, le phénomène de thermogélification étant parfaitement réversible. Un tel polymère thermogélifiant comprend, d'une part, des unités hydrosolubles et, d'autre part, des unités présentant dans l'eau une température inférieure critique de démixtion (appelée en abrégé « LCST » pour « lower critical solution température »). En dessous de la LCST, le polymère est parfaitement soluble dans l'eau. En dessus de la LCST, les unités à LCST s'associent par phénomène de réticulation entre les chaînes polymères et perdent leur solubilité dans l'eau. De manière plus précise, le polymère comprend des chaînes linéaires de type polyoxyalkylène triblocs thermosensibles constituée de blocs de polyoxyde d'éthylène (POE) et de blocs de polyoxyde de propylène (PPO), lesdites chaînes étant de la forme POE-PPO-POE, ledit polymère comprenant des enchaînements constitués d'au moins une chaîne POE-PPO-POE allongée à au moins une de ses extrémités par un groupement organique via une liaison carbamate et des enchaînements constitués d'au moins une chaîne POE-PPO-POE allongée à au moins une de ses extrémités par un groupement organique via une liaison urée.

Préférentiellement, la chaîne POE-PPO-POE répond à la formule I ci-dessous : 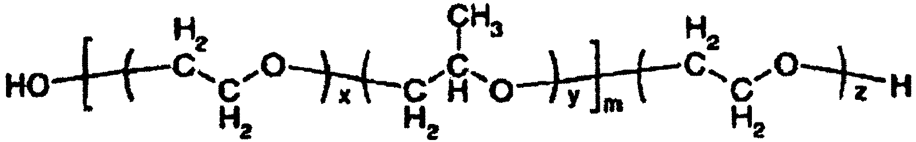 dans laquelle 20<x<120, 20<y<120, 20<z<120 et m>0,

Un tel polymère est commercialisé par la société PolymerExpert, Pessac, France, sous le nom commercial d'ExpertGel^{®} EG56.

Il a été observé qu'un tel polymère permettait d'améliorer l'application et la tenue de la composition selon l'invention sur la peau. En effet, le polymère est dit « thermo-gélifiant » et permet de former des gels à une température supérieure à 20°C. Lors de l'application de la composition sur la peau, il permet aux gouttelettes de gaz de se déformer par allongement, du fait de la température de la peau qui se trouve aux alentours de 33°C. Les gouttelettes de gaz se répartissent ainsi facilement entre les irrégularités à la surface de la peau. Le comblement des irrégularités est donc amélioré.

Préférentiellement, la composition comprend entre 0,5 et 4 % dudit polymère, en poids par rapport au poids total de la composition.

A titre de gélifiants hydrophiles autres que les polymères décrits ci-dessus, on peut citer par exemple les polymères carboxyvinyliques tels que les carbopols, les dérivés cellulosiques tels que l'hydroxyéthylcellulose, les polysaccharides et notamment les gommes telles que la gomme de xanthane ; et leurs mélanges.

Selon un mode de réalisation de l'invention, la phase grasse comprend, de plus, au moins une cire. La cire peut être présente à hauteur de 1 à 5 %, en poids.

Par cire, au sens de la présente invention, on entend un composé lipophile, solide à température ambiante, à changement d'état solide/liquide réversible, ayant un point de fusion supérieur ou égal à 30°C pouvant aller jusqu'à 120°C. Les cires peuvent être hydrocarbonées, fluorées, siliconées et être d'origine végétale, minérale, animale et/ou synthétique. Préférentiellement les cires ne sont pas siliconées. Les cires sont par exemple la cire d'abeille, la cire de lanoline, la cire de carnauba, la cire de candelilla, la cire d'Ouricuri, la cire d'Alfa, la cire de fibre de liège, la cire de canne à sucre, la cire du Japon et la cire de sumac et les cires hydrocarbonées d'une manière générale, la cire de montan, les cires microcristallines, les paraffines, la tribéhénine et l'ozokérite; les cires de polyéthylène, les copolymères cireux ainsi que leurs esters. Préférentiellement, il s'agira de la cire de trébéhénine.

Selon un autre mode de réalisation de l'invention, la composition comprend des pigments minéraux ou organiques. Comme pigments, on peut utiliser les oxydes métalliques comme les oxydes de fer, les dioxydes de titane, l'oxyde de cérium, l'oxyde de zirconium, l'oxyde de chrome. On peut utiliser également la nacre, le mica recouvert de titane ou d'oxychlorure de bismuth, les pigments nacrés colorés, ou tout autre pigment compatible avec une composition cosmétique destinée à être appliquée sur la peau. Le gaz contenu dans les gouttelettes étant hydrophobe, celles-ci vont venir tapisser la surface non traitée des pigments sectionnés et augmenter ainsi leur affinité pour la phase dispersante tout en empêchant leur regroupement.

Avantageusement, la composition, prend la forme d'une crème, d'un fond de teint ou d'un anti-cernes, préférentiellement d'un fond de teint.

Comme il sera facilement apprécié par l'homme du métier, la composition de l'invention peut contenir également des adjuvants habituels dans le domaine cosmétique, tels que les solvants, les conservateurs, les principes actifs, les humectants, les antioxydants, les agents complexants, les parfums, les bactéricides, les filtres, les agents régulateurs de pH, les colorants. Les quantités de ces différents adjuvants sont celles classiquement utilisées dans le domaine, et par exemple de 0,01 à 25% du poids total de la composition. Ces adjuvants, selon leur nature, peuvent être introduits dans la phase grasse et/ou dans la phase aqueuse.

La présente invention concerne encore un procédé de préparation d'une composition telle que définie précédemment, le procédé comprenant les étapes de :
- Préparation d'une phase grasse par mélange des composés lipophiles, dont au moins une huile de silicone et au moins un tensioactif siliconé de HLB ≤ 10,
- Préparation d'une première phase aqueuse par mélange des composés hydrophiles,
- Mélange de ladite phase grasse avec ladite première phase aqueuse de manière à former une émulsion de type eau-dans-silicone,
- Mélange d'une seconde phase aqueuse et d'un système stabilisant comprenant au moins un tensioactif de HLB ≥ à 18 et préférentiellement au moins un composé gélifiant hydrophile, puis introduction d'un gaz dans ce mélange de manière à former une phase gaz-dans eau, dite phase mousse,
- Mélange de la phase mousse et de l'émulsion de type eau-dans-silicone.

L'introduction du gaz par action mécanique est par exemple réalisée par battage ou à l'aide d'un dispositif d'aération par injection ou sur-pression d'un gaz et de cisaillement, par exemple à l'aide d'un dispositif rotor/stator ou d'un foisonneur, par exemple à l'aide d'un foisonneur de type Mondomix, d'un batteur de type Kenwood, d'un échangeur à surface raclée ou d'un mélangeur dynamique ou d'un dispositif statique, par exemple SMX (Koch-Sulzer) ou Kenics (Chemineer) ou encore foisonnement par membrane.

De manière alternative, l'introduction du gaz est réalisée par action chimique à l'aide d'un composé apte à libérer un gaz tel que du bicarbonate de sodium. Dans ce mode de réalisation, l'ajout du composé chimique directement dans la composition permet de générer des gouttelettes de gaz. L'ajout peut se faire dans une proportion d'environ 2% de composé apte à libérer un gaz, par rapport au poids total de l'émulsion.

La seconde phase aqueuse, avant introduction du gaz, présente une viscosité comprise entre 4 et 10 Pa.s (pascal.seconde), préférentiellement environ 8 Pa.s. La viscosité est mesurée à l'aide d'un rhéomètre MCR 502 (Société Anton Paar) à géométrie plan-plan (diamètre : 25 mm ; Gap : 0,6 mm) dans des conditions de mesure dans lesquelles la température est de 25 °C et le taux de cisaillement est de 10 s⁻¹ .

La seconde phase aqueuse, après introduction du gaz, c'est à dire la phase mousse, présente une viscosité comprise entre 7 et 12 Pa.s (pascal.seconde), préférentiellement environ 10 Pa.s. (rhéomètre MCR 502 (Société Anton Paar) à géométrie plan-plan (diamètre : 25 mm ; Gap : 0,6 mm) dans des conditions de mesure dans lesquelles la température est de 25 °C (le taux de cisaillement est de 10 s⁻¹).

Avantageusement, la composition selon l'invention présente, après mélange de la phase mousse et de l'émulsion de type eau-dans-silicone, une viscosité comprise entre 10 et 18 Pa.s, préférentiellement 15 Pa.s (rhéomètre MCR 502 Anton Paar à géométrie plan-plan, diamètre: 25 mm; Gap: 0,6 mm; température de 25 °C et le taux de cisaillement de 10 s⁻¹).

Les caractéristiques de l'invention mentionnées ci-dessus, ainsi que d'autres, apparaîtront plus clairement à la lecture de la description suivante d'un exemple de réalisation.

### EXEMPLE

Une composition cosmétique de fond de teint est réalisée selon la formulation illustrée dans le Tableau 1.

Les composés de la phase grasse sont pesés un à un. La préparation est chauffée à 65°C sous agitation afin d'homogénéiser. Les pigments et poudres ainsi que le gel de cyclopentasiloxane, carbonate de propylène et disteardimonium hectorite sont ajoutés sous Silverson^{®}. La phase grasse est ensuite fouettée pendant 1 min à 30°C avec une vitesse comprise entre 300 et 375 tr.min⁻¹. Les composés de la phase aqueuse sont ajoutés un à un et homogénéisés sous agitation à 82/85°C. Après ajout de l'éthylparabène, le mélange est refroidi à 55°C. Les matières suivantes sont ajoutées une à une. Après ajout de la glycérine, le mélange est refroidi à 30°C. De l'eau s'évapore pendant le chauffage, il est donc nécessaire de faire un qsp en eau. L'alcool est ajouté juste avant émulsion.

La phase mousse ou émulsion de gouttelettes de gaz correspond à une deuxième phase aqueuse fouettée sous pression entre 0.1 et 6 bars au dessus de la pression atmosphérique. Le tensioactif PEG-150 Distéarate et le gélifiant polyacrylate de sodium sont introduits un à un et homogénéisés à 70°C sous agitation. On réalise le qsp en eau. Le mélange est fouetté à 60°C pendant 1 min avec une vitesse comprise entre 100 et 375 tr.min⁻¹ afin d'introduire le gaz dans cette phase sous une pression de 1 bar minimum.

Par la suite, l'émulsion de la phase aqueuse dans la phase grasse est réalisée, à 30° C. La phase aqueuse est versée sous forme d'un fin filet pour favoriser le cisaillement des gouttelettes entre 10 s-1 et 7000 s-1. On émulsionne pendant 2 min sous une pression minimale de 1.5 bars dans la cuve de mélange.

La phase mousse est ajoutée lentement dans le mélange à 30°C sous une agitation comprise entre 100 et 375 tr.min⁻¹. Après l'ajout, l'agitation se poursuit pendant 1 min à une vitesse de 150 tr.min-1 pour un cisaillement de 1500 s-1 et sous une pression minimale de 1.5 bars dans la cuve.

**Tableau 1 : Formulation d'un fond de teint**

| **Phases** | **Nom** | **Fonction** | **Pourcentage en poids % minimum / % moyen de préférence / % maximum** |
|---|---|---|---|
| **Phase grasse** | Tribéhénine | Cire | 1/3/ 2 |
| | Cyclohexasiloxane & Cyclopentasiloxane | Solvant | qsp 100/6,7/qsp 100 |
| | Hexyl laurate & Polyglyceryl-` isostearate & Pentaerythrityl tetra-di-t-butyl hydroxyhydrocinnamate & Cetyl PEG/PPG-10/1 diméthicone | Emulsionnant HLB =5 | 1/1,5/4 |
| | Cyclopentasiloxane & PEG/PPG-19/19 diméthicone & Tocophérol | Emulsionnant HLB =3,5 | 1/3/6 |
| | Phenyl triméthicone | Emollient | 4,5/5,5/7 |
| | Acétate de tocophérol | Antioxydant | 0,2/0,5/0,8 |
| | Palmitate de rétinol & Tocophérol | Antioxydant | 0/0,07/0,1 |
| | Ethylhexyle méthoxycinnamate & BHT | Filtre solaire | 0/5/5 |
| | CI 77891 & Triéthoxycaprylylsilane | Pigment | 2/8,88/15 |
| | CI 77492 & Triéthoxycaprylylsilane | Pigment | 0,5/1,14/3 |
| | CI 77491 & Triéthoxycaprylylsilane | Pigment | 0,1/0,42/2 |
| | CI 77499 & Triéthoxycaprylylsilane | Pigment | 0,1/ 0,34/0,6 |
| | Cyclométhicone & Méthacryate de lauryle/glycol dimethacrylate crosspolymer | Poudre matifiante | 0,5/1,42 /3 |
| | Oxyde de zinc | Poudre matifiante | 0/1,35/3 |
| | Polyamide-5 | | 1/3,48/6 |
| | Cyclopentasiloxane & Carbonate de propylène & Disteardimonium hectorite | Gel (viscosité) | 4/8/10 |
| | Palmitate d'isopropyle & Lécithine & Acide stéarique & Hydroxide d'aluminium & Dioxyde de titane | Filtre solaire | 0/1,29/3 |
| **Phase aqueuse** | Eau | Solvant | 4/9,49/15 |
| | Phénoxyéthanol | Conservateur | 0,15 |
| | Sulfate de magnésium & Eau | Agent stabilisant | 0,1/0,58/1 |
| | Méthylparabène | Conservateur | 0,03 |
| | Ethylparabène | Conservateur | 0,02 |
| | Eau & Extrait de pépins de lentilles & Extrait *d'Evernia futuracea* & Extrait *d'Evernia prunastri &* | Actif astringent | 1,24 |
| | Acide salicylique | Conservateur | 0/0,25/3 |
| | Solution aqueuse d'hydroxyde de potassium à 50% | Agent de pH | 0,18 qsp pH :7-8 |
| | Glycérine | Humectant | 1/3/6 |
| | Ethanol | Solvant, antimicrobien Antimousse | 1/3/5 |
| **Phase mousse** | Eau | Solvant | 15/29,29/35 |
| | PEG-150 Distéarate | Tensioactif HLB = 18,4 | 0,8/1/5 |
| | Polyacrylate de sodium | Gélifiant | 0,01/0,18/1 |

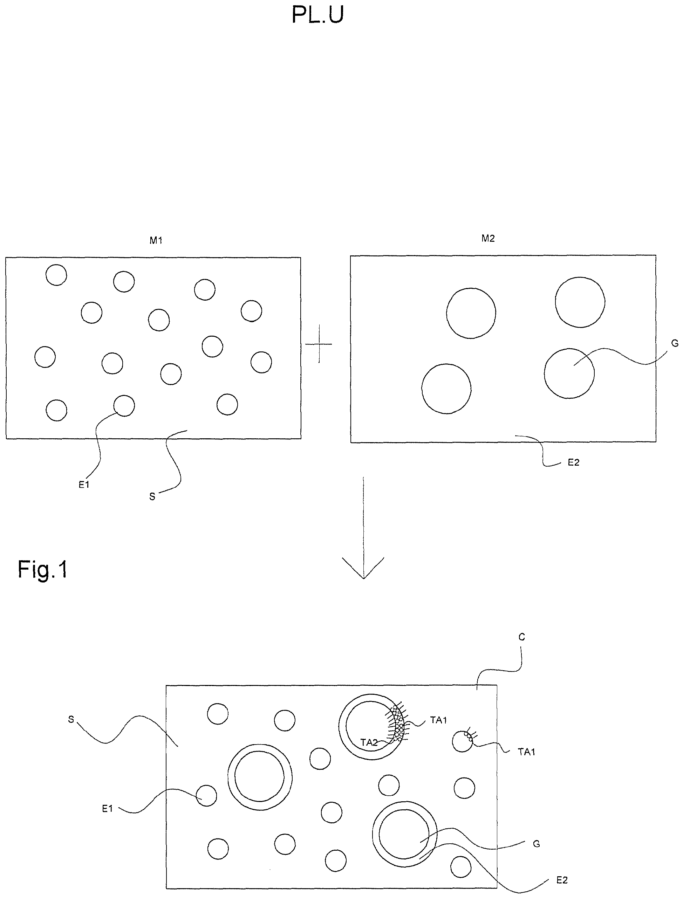

Sur la Fig. 1 est représentée de manière schématique la mise en place de la structure interne de la composition formulée : lorsque la phase mousse M2 est formulée, on observe une dispersion de gouttelettes de gaz G dans le solvant aqueux E2, de l'eau dans cet exemple.

Lorsque l'émulsion de type eau-dans silicone M1 est formulée, on observe une dispersion de gouttelettes de solvant aqueux E1, de l'eau dans cet exemple, dans la phase grasse siliconée continue S. Lorsque les deux émulsions M1 et M2 sont mélangées, on observe dans la composition C obtenue une dispersion de gouttelettes d'eau E1 dans la phase grasse continue S cohabitant avec une dispersion de gouttelettes de gaz G entourées chacune d'un film d'eau E2. La structure contient ainsi deux émulsions : une émulsion eau-dans-huile et une émulsion gaz-dans-eau-dans huile.

Les tensioactifs TA2, présentant un HLB de 18,4 se trouvent dans la phase mousse M2, leur partie hydrophobe tournée vers le gaz. Ils prennent leur place lors du fouettage de cette phase autour des gouttelettes de gaz. Lors de l'introduction de cette phase dans l'émulsion M1, les tensioactifs TA1 se trouvant dans la phase grasse (HLB = 5 et 3,5) se placent à la fois autour des gouttelettes d'eau E1 de l'émulsion eau/silicone autour des gouttelettes d'eau E2 qui renferment du gaz.

Un réseau de gel est créé par le sodium polyacrylate dans la phase mousse M2. Des liaisons hydrogènes s'établissent entre les molécules d'eau et les groupes carboxylates présents sur les chaînes polymères, et entre les molécules d'eau et le tensioactif de HLB ≥16 (TA2).

Dans la composition de fond de teint C, un réseau de gel maintient les gouttelettes de gaz G. Celles-ci sont stabilisées par les tensioactifs de HLB ≤ 10 et les gélifiants.

### Stabilité du fond de teint :

Afin de juger la stabilité du fond de teint, des centrifugations et des tests de screening ont été réalisés. Des échantillons de fond de teint ont été conservés à 5°C, 40°C et 50°C pendant 3 mois. Des prélèvements toutes les semaines pendant un mois puis tous les mois ont été réalisés et analysés de façon organoleptique (odeur, couleur, aspect). Le fond de teint est stable, les gouttelettes de gaz restent dans la composition.

Une fois appliquée à la surface de la peau, on observe au microscope que les creux et irrégularités sont comblés.

### EXEMPLE 2

Dans cet exemple, la formule 2 est basée sur celle donnée dans le Tableau 1 à part qu'un polymère thermosensible ExpertGel^{®} EG56 a été ajouté dans la phase mousse à une teneur de 2% (poids). La formule 2 est stable selon les mêmes critères que ceux décrits dans l'exemple 1. De plus, la composition une fois appliquée sur la peau peut être ôtée à l'aide d'eau tiède simplement ce qui facilite le démaquillage. En outre, l'adhésion sur le derme et le comblement des creux et irrégularités à la surface sont renforcés visiblement (observation à l'aide d'un microscope)

## Revendications

1. Composition cosmétique de soin et/ou de maquillage de la peau sous la forme d'une émulsion de type eau-dans-silicone, de densité inférieure à 1,1, et comprenant, en poids, par rapport au poids de la composition :
- entre 40 et 90 % d'une phase grasse siliconée continue comprenant au moins une huile siliconée et entre 1 et 4% de cetyl PEG/PPG-10/1 diméthicone et entre 1 et 6% de cyclopentasiloxane et PEG/PPG- 19/19 diméthicone, à titre de tensioactifs siliconés de HLB inférieur ou égal à 10,
- une phase aqueuse dispersée dans ladite phase grasse continue,
- une dispersion de gouttelettes de gaz de diamètre moyen compris entre 10 nm et 1 µm,
- un système stabilisant pour stabiliser la dispersion de gouttelettes de gaz comprenant entre 0,8 et 5% de PEG-150 distéarate à titre de tensioactif de HLB supérieur ou égal à 16 et entre 0,01 et 1% de polyacrylate de sodium à titre de composé gélifiant hydrophile.

2. Composition selon la revendication 1, **caractérisée en ce que** la dispersion de gouttelettes de gaz se présente sous la forme d'une dispersion gaz-dans-eau-dans silicone.

3. Composition selon la revendication 1 ou 2, **caractérisée en ce que** ledit gélifiant hydrophile comprend un polymère thermogélifiant, en particulier un polymère qui comprend des chaînes linéaires de type polyoxyalkylène triblocs thermosensibles constituées de blocs de polyoxyde d'éthylène (POE) et de blocs de polyoxyde de propylène (PPO), lesdites chaînes étant de la forme POE-PPO-POE, ledit polymère comprenant des enchaînements constitués d'au moins une chaîne POE-PPO-POE allongée à au moins une de ses extrémités par un groupement organique via une liaison carbamate et des enchaînements constitués d'au moins une chaîne POE-PPO-POE allongée à au moins une de ses extrémités par un groupement organique via une liaison urée.

4. Composition selon l'une des revendications précédentes, **caractérisée en ce que** ladite phase grasse comprend, de plus, au moins une cire, préférentiellement la cire de tribéhénine.

5. Composition selon l'une des revendications précédentes, **caractérisée en ce qu'**elle comprend entre 1 et 5 % de cire.

6. Composition selon l'une des revendications précédentes, **caractérisée en ce qu'**elle comprend, de plus au moins un pigment, au moins un colorant, des conservateurs, des poudres, des filtres solaires, des principes actifs ou des agents de pH.

7. Composition selon l'une des revendications précédentes, **caractérisée en ce qu'**elle se présente sous la forme d'une crème, d'un fond de teint ou d'un anticerne, préférentiellement d'un fond de teint.

8. Procédé de préparation d'une composition telle que définie dans l'une des revendications 1 à 7, **caractérisé en ce qu'**il comprend les étapes de :
- mélange d'entre 40 et 90 % d'une phase grasse siliconée comprenant au moins une huile siliconée et d'entre 0,1 et 8 % de cetyl PEG/PPG-10/1 diméthicone et de cyclopentasiloxane et PEG/PPG- 19/19 diméthicone avec entre 23 et 73 % d'une phase aqueuse de manière à former une émulsion de type eau-dans-silicone,
- mélange d'eau et d'un système stabilisant comprenant entre 0,001 et 2 % de PEG-150 distéarate et entre 0,001 et 4 % de polyacrylate de sodium, puis introduction d'air dans ce mélange de manière à former une phase air-dans eau, dite phase mousse,
- mélange de la phase mousse et de l'émulsion de type eau-dans-silicone.

## Patentansprüche

1. Kosmetikzusammensetzung zur Pflege und/oder zum Schminken der Haut in Form einer Emulsion des Wasser-in-Silikon-Typs mit einer Dichte von weniger als 1,1, die in Gew.-%, bezogen auf das Gewicht der Zusammensetzung, Folgendes umfasst:
- zwischen 40 und 90 % einer kontinuierlichen Silikon-Fettphase, die mindestens ein Silikonöl und zwischen 1 und 4 % Cetyl-PEG/PPG-10/1 Dimethicone und zwischen 1 und 6 % Cyclopentasiloxan und PEG/PPG-19/19 Dimethicone als silikonhaltige Tenside mit einem HLB-Wert von weniger als oder gleich 10 umfasst,
- eine wässrige Phase, die in der kontinuierlichen Fettphase dispergiert ist,
- eine Dispersion von Gaströpfchen mit einem mittleren Durchmesser zwischen 10 nm und 1 µm,
- ein Stabilisierungssystem zum Stabilisieren der Dispersion von Gaströpfchen, das zwischen 0,8 und 5 % PEG-150-distearat als Tensid mit einem HLB-Wert von mehr als oder gleich 16 und zwischen 0,01 und 1 % Natriumpolyacrylat als hydrophile gelbildende Verbindung umfasst.

2. Zusammensetzung nach Anspruch 1, **dadurch gekennzeichnet, dass** die Dispersion von Gaströpfchen in Form einer Gas-in-Wasser-in-Silikon-Dispersion vorliegt.

3. Zusammensetzung nach einem der Ansprüche 1 oder 2, **dadurch gekennzeichnet, dass** der hydrophile Gelbildner ein wärmegelierendes Polymer umfasst, insbesondere ein Polymer, das gerade Ketten des Typs wärmeempfindlichen Triblock-Polyoxyalkylens umfasst, die aus Blöcken von Polyethylenoxid (POE) und Blöcken von Polypropylenoxid (PPO) bestehen, wobei die Ketten die Form POE-PPO-POE haben, wobei das Polymer Aneinanderreihungen, die aus mindestens einer POE-PPO-POE-Kette bestehen, die an mindestens einem ihrer Enden durch eine organische Gruppe über eine Carbamatbindung verlängert ist, und Aneinanderreihungen, die aus mindestens einer POE-PPO-POE-Kette bestehen, die an mindestens einem ihrer Enden durch eine organische Gruppe über eine Harnstoffbindung verlängert ist, umfasst.

4. Zusammensetzung nach einem der vorhergehenden Ansprüche, **dadurch gekennzeichnet, dass** die Fettphase außerdem mindestens ein Wachs, vorzugsweise Tribehenin-Wachs, umfasst.

5. Zusammensetzung nach einem der vorhergehenden Ansprüche, **dadurch gekennzeichnet, dass** sie zwischen 1 und 5 % Wachs umfasst.

6. Zusammensetzung nach einem der vorhergehenden Ansprüche, **dadurch gekennzeichnet, dass** sie außerdem mindestens ein Pigment, mindestens einen Farbstoff, Konservierungsmittel, Puder, Sonnenschutzmittel, Wirkstoffe oder pH-Mittel umfasst.

7. Zusammensetzung nach einem der vorhergehenden Ansprüche, **dadurch gekennzeichnet, dass** sie in Form einer Creme, einer Grundierung oder eines Abdeckstifts, vorzugsweise einer Grundierung, vorliegt.

8. Verfahren zur Herstellung einer Zusammensetzung nach einem der Ansprüche 1 bis 7, **dadurch gekennzeichnet, dass** es die folgenden Schritte umfasst:
- Mischen von zwischen 40 und 90 % einer Silikon-Fettphase, die mindestens ein Silikonöl und zwischen 0,1 und 8 % Cetyl-PEG/PPG-10/1 Dimethicone und Cyclopentasiloxan und PEG/PPG-19/19 Dimethicone umfasst, mit einer wässrigen Phase zur Herstellung einer Emulsion des Wasser-in-Silikon-Typs,
- Mischen von Wasser und einem Stabilisierungssystem, das zwischen 0,001 und 2 % PEG-150-distearat und zwischen 0,001 und 4 % Natriumpolyacrylat umfasst, dann Einbringen von Luft in dieses Gemisch zur Herstellung einer Luftin-Wasser-Phase, einer so genannten Schaumphase,
- Mischen der Schaumphase und der Emulsion des Wasser-in-Silikon-Typs.

## Claims

1. Cosmetic composition for caring for and/or making up the skin, in the form of an emulsion of water-in-silicone type, having a density of less than 1.1, and comprising, by weight relative to the weight of the composition:
- between 40 and 90% of a continuous silicone fatty phase comprising at least one silicone oil and between 1 and 4% of cetyl PEG/PPG-10/1 dimethicone and between 1 and 6% of cyclopentasiloxane and PEG/PPG-19/19 dimethicone, as silicone surfactants having an HLB of less than or equal to 10,
- an aqueous phase dispersed in said continuous fatty phase,
- a dispersion of gas droplets having a mean diameter of between 10 nm and 1 µm,
- a stabilizing system for stabilizing the dispersion of gas droplets, comprising between 0.8 and 5% of PEG-150 distearate as surfactant having an HLB of greater than or equal to 16 and between 0.01 and 1% of sodium polyacrylate as hydrophilic gelling compound.

2. Composition according to Claim 1, **characterized in that** the dispersion of gas droplets is in the form of a gas-in-water-in-silicone dispersion.

3. Composition according to one of Claims 1 or 2, **characterized in that** said hydrophilic gelling agent comprises a thermogelling polymer, in particular a polymer which comprises linear heat-sensitive triblock polyoxyalkylene-type chains constituted of polyethylene oxide (POE) blocks and polypropylene oxide (PPO) blocks, said chains being of the form POE-PPO-POE, said polymer comprising sequences constituted of at least one POE-PPO-POE chain extended at at least one of its ends by an organic group via a carbamate bond and sequences constituted of at least one POE-PPO-POE chain extended at at least one of its ends by an organic group via a urea bond.

4. Composition according to one of the preceding claims, **characterized in that** said fatty phase additionally comprises at least one wax, preferentially tribehenin wax.

5. Composition according to one of the preceding claims, **characterized in that** it comprises between 1 and 5% of wax.

6. Composition according to one of the preceding claims, **characterized in that** it additionally comprises at least one pigment, at least one colorant, preservatives, powders, sunscreens, active principles or pH agents.

7. Composition according to one of the preceding claims, **characterized in that** it is in the form of a cream, foundation or concealer, preferentially a foundation.

8. Process for preparing a composition as defined in one of Claims 1 to 7, **characterized in that** it comprises the steps of:
- mixing between 40 and 90% of a silicone fatty phase comprising at least one silicone oil and between 0.1 and 8% of cetyl PEG/PPG-10/1 dimethicone and cyclopentasiloxane and PEG/PPG-19/19 dimethicone with an aqueous phase so as to form an emulsion of water-in-silicone type,
- mixing water and a stabilizing system comprising between 0.001 and 2% of PEG-150 distearate and between 0.001 and 4% of sodium polyacrylate, and then introducing air into this mixture so as to form an air-in-water phase, referred to as foam phase,
- mixing the foam phase and the emulsion of water-in-silicone type.
